**Europäisches Patentamt**

**European Patent Office**

**Office européen des brevets**

(19)

(11) Veröffentlichungsnummer : **0 088 275**
**B1**

(12)

# EUROPÄISCHE PATENTSCHRIFT

(45) Veröffentlichungstag der Patentschrift :
22.01.86

(51) Int. Cl.⁴ : **C 07 C 29/38, C 07 C 31/22**

(21) Anmeldenummer : **83101625.8**

(22) Anmeldetag : **21.02.83**

(54) Verfahren zur Herstellung von Trimethylolpropan.

(30) Priorität : 04.03.82 DE 3207746

(43) Veröffentlichungstag der Anmeldung :
14.09.83 Patentblatt 83/37

(45) Bekanntmachung des Hinweises auf die Patenterteilung : 22.01.86 Patentblatt 86/04

(84) Benannte Vertragsstaaten :
BE CH DE FR GB IT LI NL SE

(56) Entgegenhaltungen :
BE-A- 467 859
DE-A- 1 643 180
DE-B- 1 182 646
DE-C- 134 514
DE-C- 733 849
GB-A- 904 780
GB-A- 1 163 428
US-A- 2 170 624
US-A- 3 076 854

(73) Patentinhaber : **BAYER AG**
**Konzernverwaltung RP Patentabteilung**
**D-5090 Leverkusen 1 Bayerwerk (DE)**

(72) Erfinder : **Immel, Otto, Dr.**
**Immenhofweg 26**
**D-4150 Krefeld (DE)**
Erfinder : **Schwarz, Hans-Helmut, Dr.**
**Rather Strasse 90**
**D-4150 Krefeld (DE)**
Erfinder : **Quast, Hein, Dr.**
**Camendonkstrasse 1**
**D-4150 Krefeld (DE)**
Erfinder : **Bandtel, Eberhard**
**Dahler Dyk 173**
**D-4150 Krefeld (DE)**

**Beschreibung**

Die Erfindung betrifft ein Verfahren zur Herstellung von Trimethylolpropan durch Umsetzung von n-Butyraldehyd mit wäßrigem Formaldehyd in Gegenwart von alkalischen Kondensationsmitteln.

Aus der DE-AS 11 53 739, der DE-AS 11 54 080 und der DE-AS 11 82 646 ist es bekannt, Trimethylolpropan durch alkalische Kondensation von Formaldehyd mit Butyraldehyd herzustellen. Nachteilig bei diesem Verfahren sind die für ein großtechnisches Verfahren unbefriedigenden Ausbeuten und der z. T. erforderliche, hohe technische Aufwand (mehrere Reaktionsstufen z. B. bei dem Verfahren der DE-AS 11 54 080 und der DE-AS 11 53 739). Aus der US-PS 3.076.854 ist ebenfalls ein Verfahren zur Herstellung von Trimethylolpropan durch alkalische Kondensation von Formaldehyd mit Butyraldehyd bekannt. Gemäß Beispiel 1 der genannten US-PS wird methanolfreier wäßriger Formaldehyd eingesetzt. Nach der Umsetzung erhält man ein Reaktionsgemisch, das Trimethylolpropan zu 15,9 % und Formaldehyd zu 0,4 % enthält. Keine näheren Angaben sind dem Beispiel 1 der US-PS 3.076.854 zu den Ausbeuten an Trimethylolpropan zu entnehmen. Unter Zugrundelegung der in dem Beispiel 1 angegebenen Mengenangaben wurden die Ausbeuten an Trimethylolpropan berechnet, was eine Ausbeute an Trimethylolpropan von 70 % der Theorie, bezogen auf eingesetzten n-Butyraldehyd, ergab. Ausbeuten von etwa 70 % der Theorie an Trimethylolpropan sind aber, wie zuvor bereits geschildert, für ein großtechnisches Verfahren nicht akzeptabel.

Aus der DD-PS 13 45 14 ist außerdem ein Verfahren bekannt, bei dem Trimethylolpropan durch alkalische Kondensation von annähernd stöchiometrischen Mengen an n-Butyraldehyd und wäßrigem Formaldehyd in Gegenwart eines Gemisches aus Calciumhydroxid und Natriumhydroxid als Kondensationsmittel sowie durch Einstellen eines Mindestwassergehaltes im Reaktionsgemisch hergestellt wird. Nachteilig bei diesem Verfahren ist, daß das als wichtiges Nebenprodukt anfallende Calciumformiat mit Natriumformiat verunreinigt ist, wobei das Natriumformiat nur mit größerem technischem Aufwand abgetrennt werden kann. Außerdem konnten bei der Nacharbeitung des in der DD-PS beschriebenen Verfahrens die dort angegebenen hohen Ausbeuten an Trimethylolpropan nicht erreicht werden (vgl. das Vergleichsbeispiel zu Beispiel 2 des erfindungsgemäßen Verfahrens).

Eine andere Möglichkeit zur Herstellung von Trimethylolpropan besteht darin, den Butyraldehyd mit Formaldehyd unter alkalischen Reaktionsbedingungen zunächst bis zum Dimethylolbutanal umzusetzen und dieses anschließend zum Trimethylolpropan katalytisch zu hydrieren (vgl. z. B. DE-PS 27 02 582 und US-PS 4.122.290). Zwar werden mit diesen Verfahren befriedigende Ausbeuten an Trimethylolpropan erreicht (gemäß der DE-PS bis zu 94,5 % der Theorie), für ein großtechnisches Verfahren ist es dennoch weniger geeignet, da die katalytische Hydrierung in einer Hochdruckanlage durchgeführt werden muß, was einen zusätzlichen technischen Aufwand erfordert.

Es wurde nun ein Verfahren zur Herstellung von Trimethylolpropan durch Umsetzung von n-Butyraldehyd mit wäßrigem Formaldehyd in Gegenwart von alkalischen Kondensationsmitteln, wobei man den n-Butyraldehyd bei Temperaturen von 15 bis 50 °C in ein Gemisch aus Wasser, alkalischen Kondensationsmitteln und Formaldehyd eindosiert und nach Zugabe des Butyraldehyds das Reaktionsgemisch auf bis zu 90 °C erwärmt, gefunden, das dadurch gekennzeichnet ist, daß man die Umsetzung mit einem Formaldehyd durchführt, der 0,1 bis 0,001 Mol Methanol, bezogen auf 1 Mol Formaldehyd enthält, und daß in dem Reaktionsgemisch so viel Wasser zugegen ist, daß nach der Reaktion das Reaktionsgemisch einen Gehalt an Trimethylolpropan von 5 bis 10 Gew.-% aufweist.

Nach dem erfindungsgemäßen Verfahren wird der Formaldehyd im allgemeinen in Form einer wäßrigen Lösung mit einem Gehalt von etwa 10 bis 40 Gew.-% Formaldehyd, bevorzugt mit einem Gehalt von 20 bis 30 Gew.-% Formaldehyd, eingesetzt. Bevorzugt enthält der eingesetzte Formaldehyd 0,001 bis 0,05 Mol Methanol, bezogen auf 1 Mol Formaldehyd.

Als alkalische Kondensationsmittel kommen die für die Aldolkondensation bekannten und üblicherweise verwendeten Basen in Betracht. Beispielsweise seien die Hydroxide und/oder Carbonate von Alkali- und/oder Erdalkalimetallen, wie Natriumhydroxy, Kaliumhydroxid, Calciumhydroxid, Bariumhydroxid und/oder Natriumcarbonat, bevorzugt Natriumhydroxid und/oder Calciumhydroxid, besonders bevorzugt Calciumhydroxid, genannt.

Im allgemeinen werden bei dem erfindungsgemäßen Verfahren die alkalischen Kondensationsmittel in einer Menge von etwa 0,5 bis 1,7 Mol je Mol n-Butyraldehyd eingesetzt.

Bei Verwendung von Alkalihydroxiden setzt man üblicherweise etwa 1 bis 1,7, bevorzugt 1,2 bis 1,6, Mol Alkalihydroxid pro Mol Butyraldehyd und bei Einsatz von Erdalkalihydroxiden etwa 0,5 bis 1, bevorzugt 0,6 bis 0,8, Mol Erdalkalihydroxid je Mol Butyraldehyd ein.

Nach dem erfindungsgemäßen Verfahren wird im allgemeinen 1 Mol Butyraldehyd mit etwa 3,0 bis 10, bevorzugt 3,1 bis 8, besonders bevorzugt 3,2 bis 7, Mol Formaldehyd umgesetzt.

Bei der erfindungsgemäßen Umsetzung ist darauf zu achten, daß in dem Reaktionsgemisch so viel Wasser zugegen ist, daß nach der Reaktion das Reaktionsgemisch einen Gehalt an Trimethylolpropan von 5 bis 10 Gew.-% aufweist. Der erforderliche Wassergehalt des Reaktionsgemisches kann dabei durch einen entsprechen Überschuß an wäßrigem Formaldehyd oder durch Zugabe von Wasser eingestellt werden.

Das erfindungsgemäße Verfahren wird üblicherweise bei Temperaturen von etwa 15 bis 90 °C durchgeführt, wobei zu Beginn der Reaktion zunächst bei niedrigen Temperaturen, bei etwa 15 bis 50 °C,

2

bevorzugt bei 20 bis 40 °C, gearbeitet wird und anschließend zur Vervollständigung der Reaktion das Reaktionsgemisch auf höhere Temperaturen, etwa 90 °C, bevorzugt 50 bis 80 °C, erwärmt wird. In welchem Temperaturbereich die Umsetzung am zweckmäßigsten durchgeführt wird, hängt u. a. von dem pH-Wert des Reaktionsgemisches ab und kann leicht durch Vorversuche ermittelt werden. Zum Beispiel arbeitet man zunächst bei 20 bis 30 °C und erwärmt das Gemisch anschließend auf 40 bis 50 °C, wenn der pH-Wert des Reaktionsgemisches etwa 11 beträgt.

Die für das erfindungsgemäße Verfahren erforderlichen Reaktionszeiten hängen stark von der Reaktionstemperatur ab und betragen im allgemeinen etwa 5 Minuten bis 2 Stunden.

Das erfindungsgemäße Verfahren kann vorteilhafterweise so durchgeführt werden, daß man den wäßrigen Formaldehyd, das gewünschte alkalische Kondensationsmittel und gegebenenfalls Wasser vorlegt und den n-Butyraldehyd bei Temperaturen von 15 bis 50 °C eindosiert. Es ist jedoch auch möglich, nur einen Teil des Formaldehyds vorzulegen und den Rest des Formaldehyds zusammen mit dem Butyraldehyd zuzugeben. Dabei wird der Butyraldehyd zweckmäßigerweise in dem Maße dem vorgelegten Gemisch zugesetzt, wie er verbraucht wird. Nach Zugabe des Butyraldehyds erwärmt man das Reaktionsgemisch zur Vervollständigung der Reaktion auf etwa 80 °C.

Nach Beendigung der Reaktion wird der pH-Wert des Reaktionsgemisches, der in Abhängigkeit von der jeweils eingesetzten Base und der Menge des eingesetzten Formaldehyds etwa 7 bis 10 beträgt, durch Zugabe von Säuren, wie Ameisen-, Essig-, Schwefel- und/oder Phosphorsäure, auf einen Wert im Bereich von ca. 5 bis 7, bevorzugt 5,5 bis 6,5, eingestellt. Falls im Reaktionsgemisch ein Überschuß an Formaldehyd vorhanden ist, kann der Formaldehyd bei Temperaturen von etwa 120 bis 150 °C und einem Druck von etwa 2 bis 5 bar abdestilliert und auf diese Weise zurückgewonnen werden.

Die Aufarbeitung des Reaktionsgemisches kann in üblicher Weise durch Destillation oder Extraktion erfolgen (vgl. z. B. Ullmanns Enzyklopädie der technischen Chemie, 4. Auflage, Band 7, Seite 231 (1974)).

Das erfindungsgemäße Verfahren kann sowohl kontinuierlich als auch diskontinuierlich durchgeführt werden.

Der Vorteil des erfindungsgemäßen Verfahrens liegt in den hohen Ausbeuten an Trimethylolpropan, bezogen auf eingesetzten Butyraldehyd bzw. eingesetzten oder umgesetzten Formaldehyd. Dadurch kann das Verfahren im großtechnischen Maßstab besonders wirtschaftlich durchgeführt werden. Weiterhin werden bei dem erfindungsgemäßen Verfahren weniger Nebenprodukte als bei den bekannten Verfahren gebildet, wodurch sich die Aufarbeitung des Reaktionsgemisches und die Reinigung des Trimethylolpropans wesentlich einfacher gestaltet.

Besonders überraschend ist es jedoch, daß bei dem erfindungsgemäßen Verfahren die Ausbeuten an Trimethylolpropan, bezogen auf den eingesetzten bzw. umgesetzten Formaldehyd, sich noch steigern ließen, was bedeutet, daß die Ökonomie des Verfahrens im Hinblick auf den Formaldehyd-Verbrauch als äußerst günstig angesehen werden kann.

Trimethylolpropan ist ein Zwischenprodukt von technischer Bedeutung für die Herstellung von Weichmachern, Lackrohstoffen, Polyestern und Polyurethanen (vgl. z. B. Ullmanns Enzyklopädie der technischen Chemie, 4. Auflage, Band 7, Seite 228 und Seite 231 (1974)).

Die nachfolgenden Beispiele sollen das erfindungsgemäße Verfahren verdeutlichen ohne es jedoch auf diese Beispiele einzuschränken.

Beispiel 1

In einem 6 l Rührkolben werden 4 800 g (32 Mol) einer 20 %igen, wäßrigen Formaldehydlösung mit einem Methanolgehalt von 0,43 % (0,64 Mol) vorgelegt und 176 g (4,4 Mol) Natriumhydroxid, gelöst in 704 g Wasser, zugesetzt. Die Lösung wird auf 30 °C gehalten, wobei innerhalb 1 Stunde 300 g (4,04 Mol) n-Butyraldehyd (97 %ig) gleichmäßig zudosiert werden. Anschließend wird das Reaktionsgemisch auf 48 °C erwärmt und 1 Stunde bei dieser Temperatur gehalten. Das so gewonnene Reaktionsprodukt (5 980 g) enthält 8,56 % Trimethylolpropan (3,82 Mol) und 9,7 % Formaldehyd (19,3 Mol). Die Ausbeute an Trimethylolpropan beträgt 94,5 % bezogen auf den eingesetzten n-Butyraldehyd, und 90,3 % bezogen auf den umgesetzten Formaldehyd. Der nichtumgesetzte Formaldehyd wird durch Druckdestillation in üblicher Weise zurückgewonnen.

Vergleichsbeispiel zu Beispiel 1 (entsprechend dem Beispiel 1 der DE-AS 1 182 646 durchgeführt)

20 %iger wäßriger Formaldehyd in einer Menge von 4 800 g (32 Mol) mit einem handelsüblichen Methanolgehalt von 4,1 % (entsprechend 6,1 Mol) werden in einen Rührkolben mit einem Inhalt von 6 l gegeben und mit 176 g (4,4 Mol) Natriumhydroxid, gelöst in 700 g Wasser, versetzt. Zu dieser Lösung mit einer Anfangstemperatur von 28 °C werden im Verlauf von 1 Stunde unter Rühren 296 g (3,98 Mol) n-Butyraldehyd (97 %ig) gleichmäßig zudosiert. Aufgrund des exothermen Reaktionsverlaufes steigt die Temperatur auf 49 °C. Nach Zugabe des n-Butyraldehyds wird das Reaktionsprodukt auf 57 °C erwärmt und 1 Stunde bei dieser Temperatur belassen. Das Reaktionsprodukt (5 972 g) wird wie in Beispiel 1 analysiert. Es enthält 7,95 % Trimethylolpropan (3,54 Mol) und 9,6 % Formaldehyd (19,1 Mol). Die Ausbeute an Trimethylolpropan beträgt 88,9 %, bezogen auf den eingesetzten n-Butyraldehyd, und 82,3 %, bezogen auf den umgesetzten Formaldehyd.

3

## Beispiel 2

Zu 4 400 g einer wäßrigen Formalinlösung, die 3,4 % Formaldehyd (5 Mol) und 0,08 % Methanol (0,1 Mol) enthält, werden 200 g Calciumhydroxid gegeben und verrührt. In dieses Gemisch werden bei 30 °C 800 g Formalin, das 30 % Formaldehyd (8 Mol) und 0,7 % Methanol (0,2 Mol) enthält, innerhalb von 1 Stunde gleichmäßig eingepumpt. Gleichzeitig und ebenfalls im Verlauf von 1 Stunde werden 300 g 97 %iger n-Butyraldehyd (4,036 Mol) eindosiert. Dabei wird intensiv gerührt und die Temperatur auf 30 °C gehalten. Anschließend wird das Reaktionsgemisch (5 700 g) auf 60 °C erwärmt. Das so gewonnene Reaktionsprodukt enthält 8,62 % Trimethylolpropan (3,66 Mol), was einer Ausbeute von 90,7 % Trimethylolpropan entspricht, bezogen auf den eingesetzen Butyraldehyd, und 84,5 % der Theorie, bezogen auf den eingesetzten Formaldehyd.

Vergleichsbeispiel zu Beispiel 2 (entsprechend dem Beispiel 2 der DD-PS 134 514 durchgeführt)

In einen Rührkolben mit 6 l Inhalt wurden bei 20 °C 2 454 g (12,3 Mol) einer 15 %igen wäßrigen Formaldehydlösung mit einem handelsüblichen Methanolgehalt von 3,1 % (2,4 Mol) vorgelegt. Im Verlauf von 80 Minuten wurden 299 g (4,02 Mol) Butyraldehyd (97 %ig) und 1 830 g (1,1 Mol) einer 1,8 %igen wäßrigen Formaldehydlösung gleichmäßig zugepumpt, sowie 146 g (1,97 Mol) Calcium-hydroxid-Pulver und 40,9 ml einer 20 %igen Natronlauge (0,2 Mol) zudosiert. Dabei wurde die Reaktionstemperatur auf max. 40 °C beschränkt. Das erhaltene Reaktionsgemisch wurde nach Beendigung der Hauptreaktion 45 Minuten lang bei 65 °C gehalten. Das so gewonnene Reaktionsprodukt (4 762 g) enthielt 9,94 % Trimethylolpropan (3,53 Mol), was einer Trimethylolpropan-Ausbeute von 88,3 % entspricht, bezogen auf den eingesetzten n-Butyraldehyd, und 79 %, bezogen auf den eingesetzten Formaldehyd.

## Beispiel 3

In einem Rührkolben mit 6 l Inhalt wurden bei 25 °C 5 200 g einer 18,5 %igen wäßrigen Formaldehydlösung (32 Mol), die 0,4 % Methanol (0,65 Mol) enthält, vorgelegt und mit 200 g Calciumhydroxid-Pulver (2,7 Mol) versetzt. Unter starkem Rühren wurden im Verlauf von 1 Stunde 300 g (97 %ig) n-Butyraldehyd (4,036 Mol) gleichmäßig zudosiert, wobei die Reaktionstemperatur durch Kühlung auf 25 °C gehalten wurde. Nach Zugabe des Butyraldehyds wurde das Reaktionsgemisch auf 45 °C erwärmt und 1 Stunde lang bei dieser Temperatur gehalten. Das Reaktionsprodukt wurde analysiert. Es enthielt 8,86 % Trimethylolpropan und 10,1 % Formaldehyd, was einer Ausbeute an Trimethylolpropan von 93,4 % entspricht, bezogen auf den eingesetzten Butylraldehyd, und einer Ausbeute von 88,3 %, bezogen auf den umgesetzten Formaldehyd.

**Patentanspruch**

Verfahren zur Herstellung von Trimethylolpropan durch Umsetzung von n-Butyraldehyd mit wäßrigem Formaldehyd in Gegenwart von alkalischen Kondensationsmitteln, wobei man den n-Butyraldehyd bei Temperaturen von 15 bis 50 °C in ein Gemisch aus Wasser, alkalischen Kondensationsmitteln und Formaldehyd eindosiert und nach Zugabe des Butyraldehyds das Reaktionsgemisch auf bis zu 90 °C erwärmt, dadurch gekennzeichnet, daß man die Umsetzung mit einem Formaldehyd durchführt, der 0,1 bis 0,001 Mol Methanol, bezogen auf 1 Mol Formaldehyd enthält, und daß in dem Reaktionsgemisch so viel Wasser zugegen ist, daß nach der Reaktion das Reaktionsgemisch einen Gehalt an Trimethylolpropan von 5 bis 10 Gew.-% aufweist.

**Claim**

Process for the preparation of trimethylolpropane by reacting n-butyraldehyde with aqueous formaldehyde in the presence of alkaline condensing agents, the n-butyraldehyde being metered into a mixture of water, alkaline condensing agents and formaldehyde at temperatures of 15 to 50 °C and the reaction mixture being heated up to 90 °C after the addition of the butyraldehyde, characterised in that the reaction is carried out with a formaldehyde which contains 0.1 to 0.001 mol of methanol, based on 1 mol of formaldehyde, and in that such an amount of water is present in the reaction mixture that the reaction mixture has a content of trimethylolpropane of 5 to 10 % by weight after the reaction.

**Revendication**

Procédé pour la fabrication de triméthylolpropane par réaction du n-butyraldéhyde avec le formaldéhyde aqueux en présence d'agents de condensation alcalins, dans lequel on dose le n-butyraldéhyde à des températures de 15 à 50 °C dans un mélange d'eau, d'agents de condensation alcalins et de formaldéhyde et, après addition du butyraldéhyde, on chauffe le mélange de réaction jusqu'à 90 °C, caractérisé en ce que l'on met en œuvre la réaction avec un formaldéhyde qui contient 0,1 à 0,001 mol de méthanol, pour 1 mol de formaldéhyde, et en ce qu'il y a dans le mélange de réaction assez d'eau pour que le mélange de réaction présente après la réaction une teneur en triméthylolpropane de 5 à 10 % en poids.